# EUROPEAN PATENT APPLICATION

(11) **EP 3 260 448 A1**
(43) Date of publication of application: **27.12.2017**
(21) Application number: 17182311.5
(22) Date of filing: 02.05.2013
(51) Int. Cl.: C07D 263/34, C07C 59/215, C07C 67/28, C07C 67/31, C07C 69/06, C07C 69/716

(54) **PROCESS FOR PREPARATION OF 4-METHYLOXAZOLE-5-CARBOXYLIC ESTER**

(30) Priority: 03.05.2012 EP 12166591
(62) Divisional of application: 13720372.5
(71) Applicant: DSM IP Assets B.V., 6411 TE Heerlen (NL)
(72) Inventor: EISELE, FRANK, 4303 Kaiseraugst (CH)
(74) Representative: Kurt, Manfred

(57) **Abstract**

The present invention is related to a new process for the preparation of 4-methyloxazole-5-carboxylic ester, which is a valuable intermediate in the synthesis of pyridoxine (vitamin B₆). The present invention is also related to a new intermediate for the preparation of 4-methyloxazole-5-carboxylic ester and processes for the preparation thereof.

## Description

### TECHNICAL FIELD

The present invention is related to a new process for the preparation of 4-methyloxazole-5-carboxylic ester, which is a valuable intermediate in the synthesis of pyridoxine (vitamin B₆). The present invention is also related to a new intermediate for the preparation of 4-methyloxazole-5-carboxylic ester and processes for the preparation thereof.

### BACKGROUND OF INVENTION

Several processes for the manufacture of pyridoxine have already been described. A summary of the most important ones is to be found, e.g., in Ullmann's Encyclopedia of Industrial Chemistry, 5th edition, 1996, vol. A 27, p. 533-537. For the industrial synthesis of pyridoxine there is now used nearly exclusively the approach described by Kondratyeva, G.Y., Khim. Nauka Promst. 2, 666 (1957), in which approach the pyridine ring is obtained by a Diels-Alder reaction of oxazoles with maleic acid or its derivatives. A particularly preferred oxazole in the synthetic pathway is 5-cyano-4-methyloxazole which is generally prepared from 4-methyloxazole-5-carboxylic ester (see patent publications US 4,772,718 and EP 10697).

Patent publications US 3538110, IN 177708, US 4026901, US2009143346 etc. disclose the process for the preparation of 4-methyloxazole-5-carboxylic ester. US 3538110 discloses a common-used process which comprises reacting ethyl 2-chloro-acetoacetate (EACA) with formamide to give 4-methyloxazole-5-formic acid ethyl ester (OXE) together with additional formic acid and ammonium chloride.

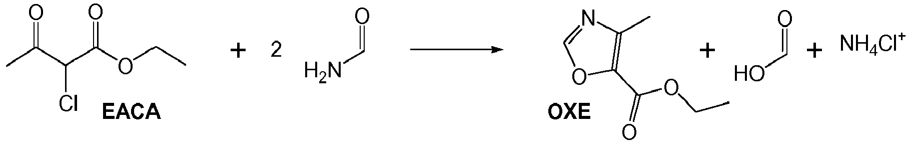

However, the process is restricted because it results in poor yields of no more than 65%. In addition, the process has the drawback of a high consumption of expensive formamide. Stoichiometrically two equivalents of formamide are needed, which can add up to 3 eq. or more under real process conditions of the process.

Therefore, there is still a existing need for a new process for the preparation of 4-methyloxazole-5-carboxylic ester.

### SUMMARY OF INENTION

The present invention provides a new process for the preparation of 4-methyloxazole-5-carboxylic ester, which has a high yield of more than 80%, and a lower cost due to low formamide consumption.

The first aspect of the present invention provides a new intermediate compound of formula (I), which can be used to prepare 4-methyloxazole-5-carboxylic ester: 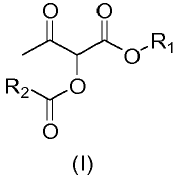 wherein each of R₁ and R₂ independently is H or C₁-C₁₀ alkyl.

The second aspect of the present invention provides a process for the preparation of 4-methyloxazole-5-carboxylic ester, represented by a compound of formula (II), which comprises reacting the compound of formula (I) with formamide in the presence of an acid catalyst, 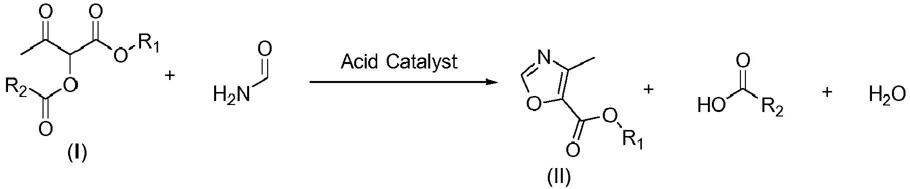 wherein R₁ and R₂ are as defined above.

The third aspect of the present invention provides a process for the preparation of the compound of formula (I) which comprises reacting a compound of formula (III) with a compound of formula (IV) in a polar solvent, 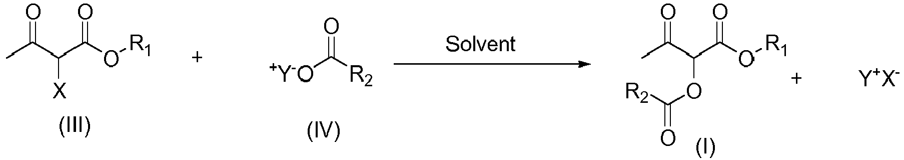 wherein:
R₁ and R₂ are as defined above;
X is halogen; and
Y is alkali metal element, alkaline-earth metal element or ammonium.

The fourth aspect of the present invention provides another process for the preparation of the compound of formula (I) which comprises reacting a compound of formula (III) with a compound of formula (IV) in a solvent in the presence of a phase transfer catalyst (PTC), 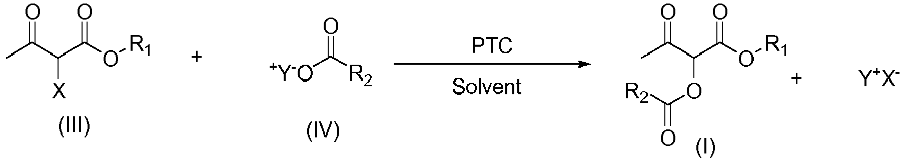 wherein R₁, R₂, X and Y are as defined above.

### DETAILED DESCRIPTION OF INVENTION

In the present invention, the term "C₁-C₁₀ alkyl" as used refers to branched or unbranched, cyclic or non-cyclic, saturated alkyl comprising 1-10 carbon atoms. Preferably, the "C₁-C₁₀ alkyl" is C₁-C₄ alkyl, and includes but is not limited to methyl, ethyl, propyl, isopropyl, cyclopropyl, butyl, isobutyl, tert-butyl, methyl cyclopropyl and cyclobutyl. More preferably, the "C₁-C₁₀ alkyl" is methyl or ethyl.

In the present invention, the term "halo" or "halogen" as used refers to a group of elements including fluorine (F), chlorine (C1), bromine (Br) and iodine (I), preferably refers to Cl or Br.

In the present invention, the term "alkali metal element" as used refers to the group 1 element excluding hydrogen in the periodic table, including lithium (Li), sodium (Na), potassium (K), rubidium (Rb), Caesium (Cs) and francium (Fr). Preferably, the term "alkali metal element" according to the present invention refers to Na or K.

In the present invention, the term "alkaline-earth metal element" as used refers to the group 2 element in the periodic table, including beryllium (Be), magnesium (Mg), calcium (Ca), strontium (Sr), barium (Ba) and radium (Ra). Preferably, the term "alkali metal element" according to the present invention refers to Mg or Ca.

The first aspect of the present invention provides a new compound of formula (I): 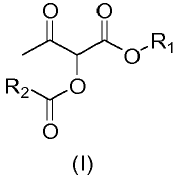 wherein each of R₁ and R₂ independently is H or C₁-C₁₀ alkyl.

Preferably, R₁ is H or C₁-C₁₀ alkyl, and R₂ is H. In an embodiment, R₁ is C₁-C₄ alkyl, and R₂ is C₁-C₄ alkyl. In another embodiment, R₁ is C₁-C₄ alkyl, and R₂ is H, methyl, ethyl, propyl or isopropyl. In a further embodiment, R₁ is methyl or ethyl; and R₂ is H.

In a preferred embodiment, the compound of formula (I) is 2-formyloxy-3-oxo-butanoic acid methyl ester, or 2-formyloxy-3-oxo-butanoic acid ethyl ester (FOXE). In the most preferred embodiment, the compound of formula (I) is FOXE.

The compound of formula (I) may be used to prepare 4-methyloxazole-5-carboxylic ester, an important intermediate for synthesis of pyridoxine.

Accordingly, the second aspect of the present invention provides a process for the preparation of 4-methyloxazole-5-carboxylic ester, represented by a compound of formula (II), which comprises reacting the compound of formula (I) with formamide in the presence of an acid catalyst, 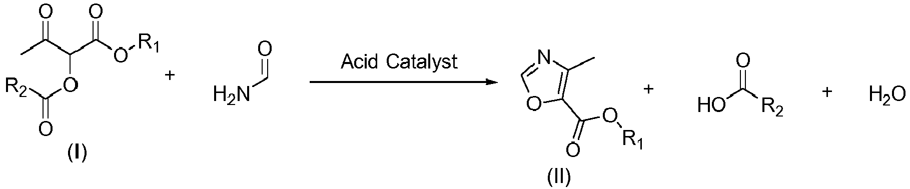 wherein R₁ and R₂ are defined as above.

In an embodiment of the above process, the compound of formula (II) is 4-methyloxazole-5-formic acid methyl ester, or 4-methyloxazole-5-formic acid ethyl ester (OXE).

The acid catalyst useful in the reaction may be any mineral acid or organic acid, preferably high boiling acids such as H₂SO₄ or H₃PO₄, or acidic ion exchange resins. The most preferred acid catalyst useful in the reaction is H₂SO₄.

Formamide may be added into the reaction in any amount. Preferably, the amount of formamide added into the reaction varies from 1.0 mol to 20 mol, more preferably it is kept between 1.0 mol and 10 mol, and most preferably between 1.0 and 2.0 mol, per 1 mol of the compound of formula (I). The amount of the acid catalyst in the reaction may vary from 1 mole% to 10 mole%, and preferably it is in the range of 3 mole% to 8 mole %, based on compound of formula (I).

The reaction may be performed batch-wise, semi-batch-wise or continuously. Preferably, carboxylic acid and water are removed from the reaction mixture during the entire reaction. For example, carboxylic acid and water are removed by distillation, such as rectification.

The reaction may be conveniently carried out under pressure and temperature conditions suitable for the reactions. Preferably, the reaction is carried out at atmospheric or reduced pressure at a temperature between 100°C and 200°C, most preferably an a temperature between 120°C and 140°C.

Optionally, the reaction is carried out under an inert gas atmosphere e.g. under nitrogen or argon or mixtures thereof. Any method known in the art, such as gas chromatography, may be used to monitor the proceeding of the reaction. After the reaction, the resulting compound of formula (II) may be used directly for synthesis of pyridoxine or isolated from the reaction by a simple straight forward method known in the art, such as filtration, distillation, rectification or combination thereof. The remaining formamide can be recycled back to the reaction, while the obtained carboxylic acid may be converted to its metal salts and then used for the preparation of the compound of formula (I), as described in the content below.

Preferably, the reaction is carried out in the absence of a solvent. In this case, the formamide consumption of the reaction can be reduced to 1.3 eq. needed for the reaction, and complex separation and recycling of formamide can be avoided. Further, as mentioned above, the carboxylic acid and water as side products of the reaction can be removed from the compound of formula (II) easily, for example, by distillation, and then recycled. Accordingly, the reaction has an extremely simple subsequent work-up and is cost efficient.

As raw material of the reaction, formamide is commercially available or may be produced by any method known in the art, and the compound of formula (I) may be prepared from 2-halo-acetoacetate by the process as described in the content below.

The third aspect of the present invention provides a process for the preparation of the compound of formula (I) from 2-halo-acetoacetate. In particular, the present invention provides a process for the preparation of the compound of formula (I) which comprises reacting a compound of formula (III) with a compound of formula (IV) in a polar solvent, 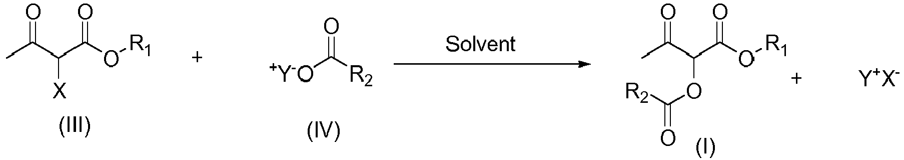 wherein:
R₁ and R₂ are as defined above;
X is halogen; and
Y is alkali metal element, alkaline-earth metal element or ammonium.

In an embodiment of the reaction, the compound of formula (III) is methyl 2-chloro-acetoacetate, or ethyl 2-chloro- acetoacetate (EACA), and the compound of formula (IV) is sodium formate or sodium acetate.

The example of the polar solvent useful in the reaction includes but is not limited to dimethylformamide (DMF) and N-methyl pyrrolidinone or mixtures thereof. The preferred solvent for the reaction is DMF. The amount of the solvent added into the reaction may be at least 1 mol, per 1 mol of the compound of formula (III).

The reaction may be carried out at reflux temperature. For example, the reaction is carried out at 153°C when DMF is used as the polar solvent or at 205°C when n-methyl pyrrolidinone is used as the polar solvent, under atmospheric pressure. The reaction may be also run at a lower reflux temperature under a reduced pressure. Preferably, the reaction is run at reduced pressure under reflux temperature between 50°C to 120°C, and more preferably 70°C to 100°C

The compound of formula (IV) may be added into the reaction in the range of 0.8 mol to 2.0 mol, preferably in the amount in the range of 0.9 mol to 1.5 mol, and more preferably in the amount in the range of 1.0 mol to 1.1 mol, per 1 mol of the compound of formula (III).

Optionally, an additional carboxylic acid may be added to enhance the reaction. Examples of the carboxylic acid are the acids with formula of R₂COOH (wherein R₂ is as defined above), including but not limit to formic acid and acetic acid. Preferably the additional carboxylic acid is added into the reaction in the range of 0.05 to 1.5 mol, more preferably in the range of 0.1 to 0.6 mol, per 1 mol of the compound of formula (III).

Optionally, the reaction is carried out under an inert gas atmosphere such as under nitrogen or argon or mixtures thereof. The reaction may be carried out as a batch procedure in the suspension of the compound of formula (IV) in the solvent or as a semi-batch procedure with the addition of the compound of formula (III). During the reaction parts of the solvent together with light boilers may be removed by, for example, rectification to achieve a complete conversion of the compound of formula (III) with a high selectivity.

Any method known in the art, such as gas chromatography, may be used to monitor the proceeding of the reaction. After the reaction, the obtained metal salt may be removed by, for example, filtration and then the solvent may be distilled off and recycled to give the crude compound of formula (I). The crude product may be purified further or used directly for the preparation the compound of formula (II).

The fourth aspect of the present invention provides another process for the preparation of the compound of formula (I) which comprises reacting a compound of formula (III) with a compound of formula (IV) in a solvent in the presence of a phase transfer catalyst (PTC), 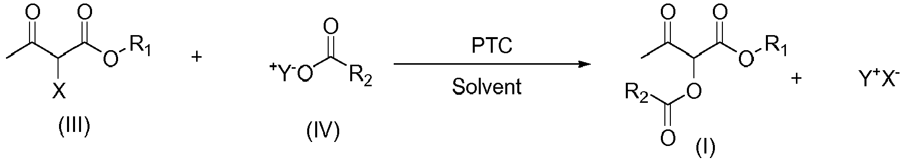 wherein R₁, R₂, X and Y are as defined above.

The solvent useful in the reaction includes but is not limited to non-polar solvents such as toluene, hexane, cyclohexane, methylcyclohexane, heptane and mixtures thereof. The most preferred solvent for the reaction is toluene.

The PTC useful in the reaction may be any quarternary ammonium salt, especially quaternary ammonium salts with one ore more non-polar alkyl chains or benzyl residues; quaternary phosphonium salt; polyglycol; or crown ether. The preferred PTCs useful in the reaction are quarternary ammonium chlorides or bromides. The most preferred PTCs useful in the reaction are tricapryl- or tri(C₈-C₁₀ alkyl)-methyl ammonium chloride, dodecyl- or hexadecyl-trimethylammonium chloride, or benzyl tributyl ammonium chloride.

The reaction may be carried out at reflux temperature. For example, the reaction is carried out at a temperature of >110°C under atmospheric pressure. The reaction may be also run at a lower temperature under a reduced pressure. Preferably, the reaction is run at temperatures between 50°C and 100°C under pressures between 200 mbar and 500 mbar.

The compound of formula (IV) may be added into the reaction in any amount. Preferably, the compound of formula (IV) is added into the reaction in the amount of 0.5 mol to 1.5 mol, more preferably in the amount of 1.0 mol to 1.5 mol, per 1 mol of the compound of formula (III). The amount of PTC used in the reaction may vary between 0.1 mole% and 10 mole% based on the compound of formula (III). In one embodiment of the process, 0.5 mol to 0.9 mol of the compound of formula (IV) and 0.1 mole% to 1.0 mole% PTC are used.

The amount of the solvent such as toluene may be at least 1 mol per 1 mol of the compound of formula (III), without affecting the selectivity of the reaction.

The process may start with the preparation of a suspension of the compound of formula (IV) in the solvent such as toluene. The suspension may be prepared by just adding the commercially available compound of formula (IV) such as sodium formate into the solvent or by adding a preparation solution of the compound of formula (IV), such as the solution from metal hydroxide and carboxylic acid, into the solvent. The suspension may be directly submitted to the reaction, or be dried and/or milled before adding into the reaction. Preferably, the suspension is dried and/or milled after the addition of the PTC.

For the reaction the above suspension may be mixed with the PTC and the starting material of the compound of formula (III). The reaction may be run in a full batch mode or semi-batch mode by adding the compound of formula (III) to the solvent/compound of formula (IV)/PTC mixture, by adding the PTC to the compound of formula (III)/ solvent/compound of formula (IV) mixture or by adding the compound of formula (IV)/solvent suspension to the compound of formula (III)/PTC/solvent mixture.

Preferably, before the raw material of the compound of formula (III) is added, the reaction mixture is set to reflux which may be achieved at normal pressure or at reduced pressure. After the addition of the compound of formula (III), the reaction mixture may be kept at reflux to remove immiscible side products (e.g. water, carboxylic acid) from the reaction mixture by, for example, distillation. These side products may be collected while the solvent may be recycled back into the reaction mixture. The reaction mixture is refluxed until the desired conversion of the compound of formula (III) is achieved.

Optionally, the reaction is carried out under an inert gas atmosphere such as under nitrogen or argon or mixtures thereof. Any method known in the art, such as gas chromatography, may be used to monitor the proceeding of the reaction. After the reaction, the obtained metal salt may be removed by filtration and then the solvent may be distilled off to give the crude compound of formula (I). The crude product may be purified further by, for example, distillation and/or rectification for the preparation the compound of formula (II).

The raw material of the compound of formula (III) is known in the art, and can be prepared by any method known in the art, such as the method disclosed in patent publications US 3538110, IN 177708, US 4026901, US2009143346. The raw material of the compound of formula (IV) can be easily prepared with carboxylic acid and metal hydroxide such as KOH and NaOH.

The whole process for preparing 4-methyloxazole-5-carboxylic ester from 2-halo-acetoacetate according to the present invention has a higher selectivity and high yield of more than 80%. In addition, the process has also other advantages including:
- Less formamide usage: in the process, less than 2.0 eq. formamide are consumed, so the process of the invention is cost efficient;
- Less organic waste: in the process, the produced carboxylic acid can be recycled and as a result of the higher yield and selectivity less organic side products are created in the reactions of the process. Also, less formamide is degraded to waste products in the reactions;
- Easy separation of inorganic salt: the metal salts produced in the reactions of the process can be precipitated easily, so less haloid waste such as chloride waste is carried into organic waste streams; and
- Less subsequent work-up: in the process, less side product is produced and less solvent is used, so the subsequent work-up is extremely simplified.

The present invention is illustrated further by the following Examples. These Examples are not intended to limit the invention in any way.

### EXAMPLES

### Example 1: Preparation of 2-formyloxy-3-oxo-butanoic acid ethyl ester (FOXE)

**1710.9** g DMF (**98**%, **22.94** mole, **8.43** eq.), **65.2** g formic acid (**99**%, **1.40** mole, **0.52** eq.) and **203.5** g sodium formate (**98**%, **2.932** mole, **1.08** eq.) were charged into a **3** L-stirred reaction vessel with a **30** mm × **30** cm Kerapak column. The mixture was dried by distillation at 70 mbar and **105**°C jacket temperature. **473.2** g EACA (**94.7**%, **2.72** mole, **1.0** eq.) was added within **10** min. The reaction mixture was reacted at 65 mbar and 85°C for **4** h while distilling off DMF/light boilers. After cooling to **24**°C, the reaction suspension was filtered to give **1911.8** g filtrate. The filter cake is washed with **316.1** g DMF. The washings are combined with the filtrate. **2272.0** g of the combined filtrate and washings are submitted to a batch distillation to remove the solvent. The resulting suspension in the sump is submitted to a filtration to obtain **441.1** g FOXE crude as the filtrate.
Overall yield of FOXE: **88.8**%
Conversion EACA: **100.0**%
Selectivity FOXE: **88.8**%

### Example 2: Preparation of FOXE with catalyst of Tricapryl-methyl ammonium chloride

In a lab ball mill, **53.0** g sodium formate (**99**%, **0.772** mole, **1.07** eq.) was suspended in **180.2** g toluene (**99**%, **1.936** mole, **2.69** eq.). The suspension was milled for **45** min at **200** rpm. The milled suspension is then transferred into the reaction vessel with stirrer and Dean-Stark-Trap. The mill was washed with **250.8** g toluene (**99**%, **2.694** mole, **3.74** eq.) and the washings were also added to the reaction vessel. **4.5** g tricapryl-methyl ammonium chloride (**70**%, **0.007** mole, **0.01** eq.) was charged to the suspension in the reaction vessel. The reaction vessel was set to a pressure of p = **361** mbar and was heated to reflux (**81**°C). The suspension was azeotropically dried, then to the stirred suspension, **129.1** g EACA (**91.7**%, **0.719** mole, **1.00** eq.) was added within **13** min. The reaction mixture was refluxed for **6** h in which a lower layer is separated in the Dean-Stark trap. After the **6** h the reaction mixture was cooled to room temperature and filtered through a nutsch filter. The filter cake was washed with **103.7** g toluene and the washings were combined with the filtrate to obtain **586.8** g FOXE solution in toluene. The toluene was distilled off and the sump FOXE can be directly submitted to the next step.
Conversion EACA: **93.0**%
Selectivity FOXE: **91.4**%
Yield FOXE: **85.0**%

### Example 3: Preparation of FOXE with catalyst of Benzyl-tri-n-butyl ammonium chloride

In a reaction vessel with stirrer and Dean-Stark-Trap, **13.0** g sodium formate **(99**%, **0.190** mole, **1.08** eq.) were suspended in **105.9** g toluene **(99**%, **1.138** mole, **6.45** eq.). **2.9** g benzyl-tri-n-butyl ammonium chloride (**98**%, **0.009** mole, **0.05** eq.) was charged into the suspension in the reaction vessel. The reaction vessel was set to a pressure of p = **361** mbar and was heated to reflux (**81**°C). The suspension was azeotropically dried, then to the stirred suspension, **31.6**g EACA **(91.7**%, **0.719** mole, **1.00** eq.) was added within **13** min. The reaction mixture was refluxed for **6** h in which a lower layer is separated in the Dean-Stark trap. After the **6** h the reaction mixture was cooled to room temperature and filtered through a nutsch filter. The filter cake was washed with **51.2** g toluene and the washings were combined with the filtrate to obtain **175.8** g FOXE solution in toluene. The toluene was distilled off and the sump FOXE can be directly submitted to the next step.
Conversion EACA: **99.0**%
Selectivity FOXE: **77.6**%
Yield FOXE: **76.8**%

### Example 4: Preparation of FOXE with catalyst of hexadecyl trimethylammonium chloride

In a reaction vessel with stirrer and Dean-Stark-Trap, **13.1** g sodium formate **(99**%, **0.190** mole, **1.08** eq.) were suspended in **106.0** g toluene **(99**%, **1.139** mole, **6.44** eq.). **3.0** g hexadecyl trimethylammonium chloride (**96**%, **0.009** mole, **0.05** eq.) was charged into the suspension in the reaction vessel. The reaction vessel was set to a pressure of p = **361** mbar and was heated to reflux (**81**°C). The suspension was azeotropically dried, then to the stirred suspension, **31.6** g EACA (**91.7**%, **0.719** mole, **1.00** eq.) was added within **15** min. The reaction mixture was refluxed for **6** h in which a lower layer is separated in the Dean-Stark trap. After the 6h the reaction mixture was cooled to room temperature and filtered through a nutsch filter. The filter cake was washed with **53.8** g toluene and the washings were combined with the filtrate to obtain **174.5** g FOXE solution in toluene. The toluene was distilled off and the sump FOXE can be directly submitted to the next step.
Conversion EACA: **98.4**%
Selectivity FOXE: **77.8**%
Yield FOXE: **76.5**%

### Example 5: Synthesis of sodium formate and then preparation of FOXE with catalyst of dodecyl trimethylammonium chloride

**271.8** g aq. NaOH (**28**%, **1.903** mole), **91.9** g formic acid (**99**%, **1.977** mole) and **55.4** g sodium formate (**98**%, **0.798** mole) were added to a stirred vessel with Dean-Stark trap. Into this aqueous sodium formate solution, **336.0** g toluene and **0.7** g dodecyl trimethylammonium chloride (**35**% solution in water, equals ca. **10**% of the complete amount later needed for the reaction) were also added. The pressure was set to p = **120** mbar and the temperature of the vessel jacket was set to **120**°C. During **6** h azeotropic removal of water was carried out resulting of a suspension of sodium formate in toluene.

In a lab ball mill, **249.2** g sodium formate/toluene suspension (from preparation above) containing **85.1** g sodium formate (**99**%, **1.239** mole, **0.70** eq.) and **164.0** g toluene (**99**%, **1.762** mole, **1.00** eq.) was added. The suspension was milled for **45** min at **200** rpm. The milled suspension was then transferred into the reaction vessel with stirrer and Dean-Stark-Trap. The mill was washed with **166.0** g toluene (**99**%, **1.784** mole, **1.01** eq.) and the washings were also added to the reaction vessel. **7.9** g dodecyl trimethylammonium chloride (**35**% in water, **0.011** mole, **0.059** eq.) was charged to the suspension in the reaction vessel. The reaction vessel was set to a pressure of p = **430** mbar and is heated to reflux (**83**°C). The suspension was azeotropically dried, then to the stirred suspension, **300.0** g EACA (**97.1**%, **1.770** mole, **1.00** eq.) were added within **15** min. The reaction mixture was refluxed for **5** h at **92**°C in which a lower layer is separated in the Dean-Stark trap. After the **5** h the reaction mixture was cooled to room temperature and filtered through a nutsch filter. The filter cake was washed with **138.0** g toluene and the washings were combined with the filtrate to obtain **714.8** g FOXE solution in toluene. The toluene was distilled off and the sump FOXE can be directly submitted to the next step.
Conversion EACA: **67.0**%
Selectivity FOXE: **90.3**%
Yield FOXE: **60.5**%

### Example 6: Recovery of EACA

To a thin film evaporator with a **50** cm × **30** mm Kerapak column at a temperature of **104**°C and at p = **5** mbar, **591.9** g EACA/FOXE mixture (**30.1**% EACA, **58.3**% FOXE) was fed continuously. At the head of the column **155.6** g recycled EACA (EACA **85.6**%) was obtained as the distillate and **11.1** g light boilers were collected in the cooling trap. As a sump, **373.2** g crude FOXE product (FOXE **87.6**%) was obtained. Also **48.7** g hold-up was collected after the experiment.
Yield EACA (Distillate, Cooling trap + hold-up): **94.5**%
Yield FOXE (Sump + Hold-up): **98.5**%

The crude FOXE product can be directly used to the next step.

### Example 7: Preparation of 4-methyloxazole-5-formic acid ethyl ester (OXE)

A **500** mL-stirred reaction vessel with a **30** mm × **30** cm Kerapak column was charged with **150.2** g formamide (**98**%, **3.267** mole, **1.60** eq.) and **13.4** g sulphuric acid (**98**%, **0.135** mole, **0.07** eq.). The mixture was heated at p = **65** mbar to an inner temperature =**120**°C. Then **400.2** g FOXE crude (**88.6**%, **2.036** mole, **1.00** eq.) was added within **5** h.. A HCOOH/water mixture was distilled from the reaction mixture during the FOXE dosage time. After the end of the FOXE dosage HCOOH/water was further distilled of to complete the reaction. This was the case after additional **1.5** h of reaction and distillation after the end of the FOXE addition. During the complete reaction an overall amount of **100.8** g HCOOH/water mixture (HCOOH **56.7**%, water **37.7**%) was distilled off. In the sump, **407.0** g OXE crude with precipitated ammonium sulphate was obtained. **400.5** g OXE crude with precipitated ammonium sulphate were filtered through a filter nutsch to get **6.0** g filter cake and **391.5** g OXE crude (OXE **68.7**%) as the filtrate. Yield in filtrate: **86.5**%

Washing of the reaction equipment with ethanol affords additional **5.4**% yield. Overall yield: **91.9** %

Washing of the filter cake was done twice with **11.0** g FOXE crude for each washing. After the washing **4.8** g filter cake which was free of OXE and **24.2** g filtrate (FOXE **76.9**%, OXE **2.0** %) were obtained. The filtrate can be recycled.
OXE yield saving: **0.6**%
Overall OXE yield from FOXE: **92.5**%
Overall OXE yield from EACA: **80.1**%

The FOXE remaining in the filter cake was washed from **4.4** g filter cake with **18.5** g toluene. After the washing, **3.9** g filter cake were obtained, which was free of FOXE. The **18.8** g filtrate (FOXE **8.6**%) can be recycled.

### Example 8: Rectification to purified OXE

To a thin film evaporator with a **50** cm × **30** mm Kerapak column at a temperature of **110**°C and at p = **5** mbar, **380.5** OXE crude was fed continuously. At the head of the column which is run at a reflux ratio of **0.2, 246.1** g OXE (OXE **89.8**%) was obtained as the distillate and as a sump **67.4** g high boiling impurities (OXE **0.6**%) was obtained. Also **37.4** g hold-up was collected after the experiment.
Loss of OXE in the sump: **0.2** %
Overall yield of OXE purified from EACA: **80.0**%

## Claims

1. A process for the preparation of the compound of formula (I) which comprises reacting a compound of formula (III) with a compound of formula (IV) in a solvent in the presence of a phase transfer catalyst (PTC), 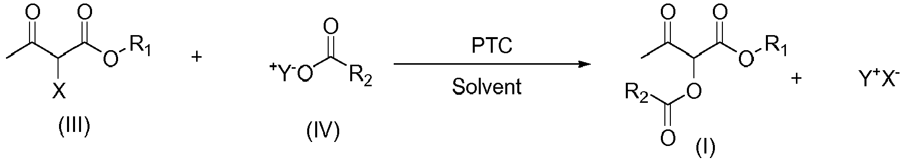 wherein:
each of R₁ is H or C₁-C₁₀alkyl; and R₂ is H;
X is halogen; and
Y is alkali metal element, alkaline-earth metal element or ammonium,
wherein the reaction is carried out at a reflux temperature.

2. The process of claim 1, wherein the solvent is a non-polar solvent such as toluene, hexane, cyclohexane, methylcyclohexane, heptane or mixtures thereof.

3. The process of claim 1 or 2, wherein the PTC is quarternary ammonium salt such as quarternary ammonium chloride or bromide selected from the group consisting of tricapryl- or tri(C₈-C₁₀alkyl)-methyl ammonium chloride, dodecyl- or hexadecyl-trimethylammonium chloride, and benzyl tributyl ammonium chloride; quaternary phosphonium salt; polyglycol or crown ether.

4. The process of any one of the claims 1-3, wherein the process starts with the preparation of a suspension of the compound of formula (IV) in the solvent.
